# EUROPEAN PATENT SPECIFICATION

(11) **EP 4 267 100 B1**
(45) Date of publication and mention of the grant of the patent: **04.02.2026**
(21) Application number: 21819150.0
(22) Date of filing: 06.12.2021
(51) Int. Cl.: A61K 8/92, A61K 8/9794, A61Q 19/08, A61K 8/9789

(54) **PERSONAL CARE COMPOSITION**
KÖRPERPFLEGEZUSAMMENSETZUNG
COMPOSITION POUR SOINS PERSONNELS

(30) Priority: 25.12.2020 WO PCT/CN2020/139550; 04.02.2021 EP 21155104
(43) Date of publication of application: 01.11.2023
(73) Proprietor: Unilever IP Holdings B.V., 6708 WH Wageningen (NL); Unilever Global IP Limited, Wirral, Merseyside CH62 4ZD (GB)
(72) Inventor: GU, Xuelan, Shanghai 200335 (CN); MI, Tingyan, Shanghai 200335 (CN)
(74) Representative: Askew, Sarah Elizabeth
(86) International application number: PCT/EP2021/084302
(87) International publication number: WO 2022/135884

(56) References cited:
- CN-A- 105 902 454
- CN-A- 106 265 456
- CN-A- 110 522 664

## Description

### Field of the invention

The present invention relates to a personal care composition comprising a moringa seed oil and a moringa extract, wherein the amount of the moringa seed oil is less than 3% by weight of the composition, the amount of the moringa extract is less than 0.3% by weight of the composition, and the weight ratio of the moringa seed oil to the moringa extract is in the range of 9:1 to 45:1. In particular such composition is capable of significantly inhibiting reactive oxygen species.

### Background of the invention

Human skin may provide physicochemical protection against environmental insults through its barrier function, mechanical strength and imperviousness to water. It is constantly exposed to harmful environments and extreme conditions, such as ultra-violet radiation, blue light, pollution etc. Such harmful environmental applications may cause excessive generation of reactive oxygen species (ROS) thereby resulting in an oxidative stress condition. Oxidative stress may affect our skin in many ways including signs of premature aging such as wrinkles, fine lines, uneven skin tone.

Therefore, a lot of efforts have been made for developing personal care composition for preventing excessive generation of reactive oxygen species, but improvements still will be required.

The present inventors have recognized there is a need to further develop a personal care composition which is able to effectively inhibit the generation of reactive oxygen species.

It was surprisingly found that a combination of low amount of moringa extract and moringa seed oil with a specific ratio exhibits a significantly better effect for inhibiting reactive oxygen species.

### Summary of the invention

In a first aspect, the present invention is directed to a personal care composition comprising a moringa seed oil and a moringa extract, as defined in claim 1.

In a second aspect, the present invention is directed to a method for providing at least one benefit selected from anti-aging, antioxidant, or oxidative stress reduction comprising a step of applying the personal care composition of the present invention.

In a third aspect, the present invention is directed to use of a personal care composition of the present invention for at least one benefit selected from anti-aging, antioxidant, or oxidative stress reduction.

All other aspects of the present invention will more readily become apparent upon considering the detailed description and examples which follow.

### Detailed description of the invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

Preferably, the moringa seed oil is extracted from seeds of at least one of *Moringa oleifera, Moringa pterygosperma, Moringa peregrina, Moringa concanensis* or *Moringa drouhardii.* The seeds of moringa are characterized by the presence of an oil whose content could vary from 20 to 80% according to the species and maturity of the seeds. For the species *Moringa oleifera,* the contents of oil range from 21 to 34% in the seeds of *Moringa oleifera.* For sake of clarity, the seed moringa oil is liquid at 25°C and atmospheric pressure.

Preferably, the moringa seed oil is extracted from the seeds of *Moringa Oleifera.* Preferably the moringa seed oil is extracted by cold-pressing. Preferably, the moringa seed oil comprises C₂₀ fatty acid, preferably behenic acid.

Preferably, the moringa seed oil is present in amount of 0.00001 to 1.5%, more preferably 0.0001 to 0.5%, even more preferably 0.004 to 0.3% and still even more preferably 0.01 to 0.1% by weight of the composition.

The moringa extract is extract of at least one selected from the group of *Moringa oleifera, Moringa pterygosperma, Moringa peregrina, Moringa concanensis* and *Moringa drouhardii.* Preferably the moringa extract comprises protein. More preferably, the moringa extract comprises 30 to 100%, even more preferably 70 to 100% of protein by weight of the moringa extract and most preferably 100% of protein by weight of the moringa extract. Preferably, the protein has a molecular weight of 2000 to 50000, preferably 5000 to 20000, measured by sodium dodecyl sulfate (SDS)-polyacrylamide gel electrophoresis. Preferably, the protein is soluble in water but insoluble in oils and fats.

The moringa extract is aqueous extract of moringa. Aqueous extract refers to that the exact is obtained by using water, or water mixing with remaining solvent. The remaining solvent may be selected from ethanol, acetone, ethyl acetate, glycerin, butylene glycol or a mixture thereof. Preferably, the aqueous extract is extract obtained by using water and/or glycerin. The moringa extract is solid at 25°C and atmospheric pressure.

Preferably, the moringa extract is present in amount of 0.000001 to 0.2%, more preferably 0.00001 to 0.05%, even more preferably 0.0002 to 0.01% and still even more preferably 0.0005 to 0.006% by weight of the composition.

Preferably, the moringa extract has an INCI name *Moringa Pterygosperma* Seed Extract. It is preferred that the moringa extract is included as PURISOFT^{®} (Ex. Laboratories Serobiologues/BASF), whose INCI name is Glycerin (and) *Moringa Pterygosperma* Seed Extract.

It is particularly preferred that the moringa seed oil and moringa extract are extracted from the same species. In such a case it is preferred that the protein and the oil are extracted from *Moringa oleifera or Moringa pterygosperma.* For sake of clarity, the moringa seed oil is different from the moringa extract.

Preferably, the weight ratio of the moringa seed oil to the moringa extract is in the range of 18:1 to 45:1, more preferably 24:1 to 45:1.

For sake of clarity, the weight of the moringa seed oil or moringa extract in the present invention typically refers to the weight of all ingredients extracted from the moringa, excluding the weight of the extract solvent.

The composition preferably comprises a cleansing surfactant. More than one cleansing surfactant may be included in the composition. The cleaning surfactant may be chosen from soap, non-soap anionic, cationic, non-ionic, amphoteric surfactant and mixtures thereof. Many suitable surface-active compounds are available and are fully described in the literature, for example, in "Surface-Active Agents and Detergents", Volumes I and II, by Schwartz, Perry and Berch. The preferred surface-active compounds that can be used are soaps, non-soap anionic, non-ionic surfactant, amphoteric surfactant or a mixture thereof.

Suitable non-soap anionic surfactants include linear alkylbenzene sulphonate, primary and secondary alkyl sulphates, particularly C₈ to C₁₅ primary alkyl sulphates; alkyl ether sulphates; olefin sulphonates; alkyl xylene sulphonates; dialkyl sulphosuccinates; fatty acid ester sulphonates; or a mixture thereof. Sodium salts are generally preferred.

Most preferred non-soap anionic surfactant are linear alkylbenzene sulphonate, particularly linear alkylbenzene sulphonates having an alkyl chain length of from C₈ to C₁₅. It is preferred if the level of linear alkylbenzene sulphonate is from 0 wt% to 30 wt%, more preferably from 1 wt% to 25 wt%, most preferably from 2 wt% to 15 wt%, by weight of the total composition.

Nonionic surfactants that may be used include the primary and secondary alcohol ethoxylates, especially the C₈ to C₂₀ aliphatic alcohols ethoxylated with an average of from 1 to 20 moles of ethylene oxide per mole of alcohol, and more especially the C₁₀ to C₁₅ primary and secondary aliphatic alcohols ethoxylated with an average of from 1 to 10 moles of ethylene oxide per mole of alcohol. Non ethoxylated nonionic surfactants include alkylpolyglycosides, glycerol monoethers, and polyhydroxyamides (glucamide). It is preferred if the level of non-ionic surfactant is from 0 wt% to 30 wt%, preferably from 1 wt% to 25 wt%, most preferably from 2 wt% to 15 wt%, by weight of a fully formulated composition comprising the microcapsules of the invention.

Suitable amphoteric surfactants preferably are betaine surfactants. Examples of suitable amphoteric surfactants include, but are not limited to, alkyl betaines, alkylamido betaines, alkyl sulfobetaines, alkyl sultaines and alkylamido sultaines; preferably, those having 8 to about 18 carbons in the alkyl and acyl group. It is preferred that the amount of the amphoteric surfactant is 0 to 20 wt%, more preferably from 1 to 10 wt%, by weight of the composition.

It is also possible to include certain mono-alkyl cationic surfactants. Cationic surfactants that may be used include quaternary ammonium salts of the general formula R¹R²R³R⁴N⁺ X⁻ wherein the R groups are long or short hydrocarbon chains, typically alkyl, hydroxyalkyl or ethoxylated alkyl groups, and X is a counter-ion (for example, compounds in which R¹ is a C₈-C₂₂ alkyl group, preferably a C₈-C₁₀ or C₁₂-C₁₄ alkyl group, R² is a methyl group, and R³ and R⁴, which may be the same or different, are methyl or hydroxyethyl groups); and cationic esters (for example, choline esters).

Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflower seed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatabilities between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

The composition may comprise water in amount of 10 to 90% by weight of the composition, more preferably from 20 to 85%, even more preferably from 25 to 78%, most preferably from 32 to 65% by weight of the composition.

Preferably, the composition has a viscosity of at least 10 mPa·s, more preferably in the range 30 to 10000 mPa·s, even more preferably 50 to 5000 mPa·s, and most preferably 100 to 2000 mPa·s, when measured at 20 degrees C at a relatively high shear rate of about 20 s⁻¹. Preferably, the composition is in the form of fluid.

Preferably, the personal care composition is a skin care composition. The term "skin" as used herein includes the skin on the face, neck, chest, abdomen, back, arms, under arms, hands, and legs. Preferably "skin" means includes the skin on the face and under arms, more preferably skin means skin on the face other than lips and eyelids.

Preferably the method is non-therapeutic. Preferably the use is non non-therapeutic. The term non-therapeutic means for cosmetic purposes and not curative or therapeutic purposes.

The present invention also provides a personal care composition comprising a moringa seed oil and a moringa extract, wherein the amount of the moringa seed oil is less than 0.8% by weight of the composition, the amount of the moringa extract is less than 0.3% by weight of the composition, and the weight ratio of the moringa seed oil to the moringa extract is in the range of 9 to 55 for use in providing at least one benefit selected from anti-aging, antioxidant, or oxidative stress reduction, particularly due to light irradiation.

The present invention also provides use of a combination of less than 0.8% of moringa seed oil by weight, less than 0.3% of moringa extract by weight wherein the weight ratio of the moringa seed oil to the moringa extract is in the range of 9 to 55 for usein the manufacture of a medicament for providing at least one benefit selected from anti-aging, antioxidant, or oxidative stress reduction

The following examples are provided to facilitate an understanding of the invention. The examples are not intended to limit the scope of the claims.

### Examples

### Materials

| Supplier | Trade name | Abbreviation | Active | Active wt% |
|---|---|---|---|---|
| BASF | Purisoft^{®} LS9726* | LS9726 | Moringa seed extract | 1.6% |
| Naturex SA | Moringa oil (LA030075-refined)** | Moringa oil | Moringa refined seed oil | 100% |

| | | | | |
|---|---|---|---|---|
| * Purisoft^{®} LS9726 was procured from BASF Beauty Solutions France SAS. ** Moringa oil (LA030075-refined) was procured from Naturex SA, France. | | | | |

### Example 1

Normal Human Epidermal Keratinocytes (NHEKs) (Biocell, Xi'an, China) were cultured in keratinocyte culture medium and plated in 6-well plates. On the second day, the culture medium was replaced with medium containing the test samples.

The moringa oil was dissolved in DMSO (dimethyl sulfoxide) at a concentration of 100 mM to form a stock solution. The samples in Table 1 were prepared accordingly by diluting the materials (except moringa oil) and/or stock solution with PBS (phosphate-buffered saline) to the desired levels as indicated in Table 1 before use for further experiment.

After incubation in each sample for another 24 hours, the medium was replaced with PBS. The cells were then irradiated with blue light using a blue LED (PL-L 18 W/52/4P, Philips, Poland), which emits light almost entirely within the 400 to 500 nm bandwidth and peaks at 450 nm. The radiation of the LED was measured with a Blue Light Safety Detector (PMA2121) (SolarLight, PA, USA) and the total energy for the blue light irradiation is 10 J/cm². The ROS (reactive oxygen species) production was evaluated after irradiation by blue light for 30 minutes, using the DCFH-DA (2',7'-Dichlorodihydrofluorescein diacetate) dye and detected with flowcytometry (CytoFLEX, Beckman Coulter, USA). Data was acquired and analyzed using the CytExpert programme (Beckman Coulter, USA).

The results were summarised in Table 1.

**Table 1**

| Sample | Contained materials (wt%)* | | Weight ratio** of Moringa oil to Moringa seed extract | ROS (Mean Fluorescent Intensity) | Inhibition (%) |
|---|---|---|---|---|---|
| | Moringa oil | LS9726 | | | |
| Control | - | - | - | 243173.3±13032.69 | - |
| 1 | - | 0.1 | - | 123223.2±7893.47 | 49.33 |
| 2 | 0.01 | - | - | 94322.2±2272.24 | 61.21 |
| 3 | 0.01 | 0.1 | 6.66667:1 | 149858.9±1637.7 ^{a} | 38.37 |
| 4 | 0.02 | | | 167504.7±8710.24 | 31.12 |
| 5 | 0.02 | 0.1 | 13.33333:1 | **56705.8±2723.73 ^{b}** | **76.68** |
| 6 | 0.05 | - | - | 191346.6±9740.45 | 21.31 |
| 7 | 0.05 | 0.1 | 33.33333:1 | **47699.6±1583.87 ^{b}** | **80.38** |
| 8 | 0.1 | - | - | 183432.7±2215.77 | 24.56 |
| 9 | 0.1 | 0.1 | 66.66667:1 | 115322.2±2898.18 ^{a} | 52.58 |

| | | | | | |
|---|---|---|---|---|---|
| *The level refers to the level of the raw material, in weight percentage. ** The ratio refers to the ratio of actives. a: not significantly better than at least one sample containing single active (p<0.05, 3 vs. 1 or 2; 9 vs. 1 or 8). b: significantly better than both samples containing single active (p<0.05, 5 vs. 1 or 4; 7 vs. 1 or 6). | | | | | |

It is evident from the data in Table 1 that the reactive oxygen species inhibition was significantly better when combining small amounts of moringa seed oil and moringa extract in the specific ratio of the present invention.

## Claims

1. A personal care composition comprising a moringa seed oil and a moringa extract, wherein:
(a) the amount of the moringa seed oil is less than 3% by weight of the composition;
(b) the amount of the moringa extract is less than 0.3% by weight of the composition;
(c) the weight ratio of the moringa seed oil to the moringa extract is in the range of 9:1 to 45:1; wherein the weight of the moringa seed oil or moringa seed extract refers to the weight of all ingredients extracted from the moringa, excluding the weight of the extract solvent;
(d) the moringa seed oil is liquid at 25°C and atmospheric pressure and the moringa extract is solid at 25°C and atmospheric pressure;
(e) the moringa extract is aqueous extract of moringa; and
(f) the moringa seed oil is different from the moringa extract.

2. The composition according to claim 1 wherein the moringa seed oil is extracted from seeds of at least one of *Moringa oleifera, Moringa pterygosperma, Moringa peregrina, Moringa concanensis* and *Moringa drouhardii.*

3. The composition according to claim 1 or 2 wherein he moringa seed oil is present in amount of 0.00001 to 1.5%, preferably 0.004 to 0.3% by weight of the composition.

4. The composition according to any one of the preceding claims wherein the moringa extract comprises protein, preferably 30 to 100% by weight of the moringa extract.

5. The composition according to any one of the preceding claims wherein the moringa extract has an INCI name *Moringa Pterygosperma* Seed Extract.

6. The composition according to any one of the preceding claims wherein the moringa extract is present in amount of 0.000001 to 0.2%, preferably 0.0002 to 0.01% by weight of the composition.

7. The composition according to any one of the preceding claims wherein the weight ratio of the moringa seed oil to the moringa extract is in the range of 18:1 to 45:1, preferably 24:1 to 45:1.

8. The composition according to any one of the preceding claims wherein the composition may comprise water in amount of 25 to 78% by weight of the composition.

9. A method for providing at least one benefit selected from anti-aging, antioxidant, and oxidative stress reduction comprising a step of applying the personal care composition of any of claims 1 to 8.

10. Use of a personal care composition of any one of claims 1 to 8 for at least one benefit selected from anti-aging, antioxidant, and oxidative stress reduction.

## Patentansprüche

1. Körperpflegezusammensetzung, die ein Moringa-Samenöl und einen Moringa-Extrakt umfasst, wobei:
(a) die Menge an Moringa-Samenöl weniger als 3 Gew.-% der Zusammensetzung beträgt;
(b) die Menge des Moringa-Extrakts weniger als 0,3 Gew.-% der Zusammensetzung beträgt;
(c) das Gewichtsverhältnis des Moringa-Samenöls zu dem Moringa-Extrakt im Bereich von 9:1 bis 45:1 liegt, wobei sich das Gewicht des Moringa-Samenöls oder Moringa-Samenextrakts auf das Gewicht aller aus dem Moringa extrahierten Inhaltsstoffe bezieht, ausgenommen das Gewicht des Extraktionslösungsmittels;
(d) das Moringa-Samenöl bei 25°C und atmosphärischem Druck flüssig ist und der Moringa-Extrakt bei 25°C und atmosphärischem Druck fest ist;
(e) der Moringa-Extrakt ein wässriger Extrakt aus Moringa ist; und
(f) sich das Moringa-Samenöl vom Moringa-Extrakt unterscheidet.

2. Zusammensetzung nach Anspruch 1, wobei das Moringa-Samenöl aus Samen von mindestens einer der folgenden Arten extrahiert ist: Moringa oleifera, Moringa pterygosperma, Moringa peregrina, Moringa concanensis und Moringa drouhardii.

3. Zusammensetzung nach Anspruch 1 oder 2, wobei das Moringa-Samenöl in einer Menge von 0,00001 bis 1,5 Gew.-%, vorzugsweise 0,004 bis 0,3 Gew.-% der Zusammensetzung, vorhanden ist.

4. Zusammensetzung nach irgendeinem der vorhergehenden Ansprüche, wobei der Moringa-Extrakt Protein umfasst, vorzugsweise 30 bis 100 Gew.-% des Moringa-Extrakts.

5. Zusammensetzung nach irgendeinem der vorhergehenden Ansprüche, wobei der Moringa-Extrakt den INCI-Namen Moringa Pterygosperma Seed Extract hat.

6. Zusammensetzung nach irgendeinem der vorhergehenden Ansprüche, wobei der Moringa-Extrakt in einer Menge von 0,000001 bis 0,2 Gew.-%, vorzugsweise 0,0002 bis 0,01 Gew.-% der Zusammensetzung vorhanden ist.

7. Zusammensetzung nach irgendeinem der vorhergehenden Ansprüche, wobei das Gewichtsverhältnis des Moringa-Samenöls zu dem Moringa-Extrakt im Bereich von 18:1 bis 45:1, vorzugsweise von 24:1 bis 45:1 liegt.

8. Zusammensetzung nach irgendeinem der vorhergehenden Ansprüche, wobei die Zusammensetzung Wasser in einer Menge von 25 bis 78 Gew.-% der Zusammensetzung umfasst.

9. Verfahren zur Bereitstellung mindestens eines Vorteils, ausgewählt unter Anti-Aging, Antioxidans und Reduzierung von oxidativem Stress, umfassend einen Schritt des Aufbringens der Körperpflegezusammensetzung nach einem der Ansprüche 1 bis 8.

10. Verwendung einer Körperpflegezusammensetzung nach irgendeinem der Ansprüche 1 bis 8 für mindestens einen Vorteil, ausgewählt unter Anti-Aging, Antioxidans und Reduzierung von oxidativem Stress.

## Revendications

1. Composition de soins personnels comprenant une huile de graines de moringa et un extrait de moringa, dans laquelle :
(a) la quantité de l'huile de graines de moringa est inférieure à 3 % en poids de la composition ;
(b) la quantité de l'extrait de moringa est inférieure à 0,3 % en poids de la composition ;
(c) le rapport pondéral de l'huile de graines de moringa à l'extrait de moringa est compris dans la plage allant de 9:1 et 45:1 ; le rapport de l'huile de graines de moringa à l'extrait de moringa faisant référence au poids de tous les ingrédients extraits du moringa, à l'exclusion du poids du solvant d'extraction ;
(d) l'huile de graines de moringa est liquide à 25 °C et sous pression atmosphérique et l'extrait de moringa est solide à 25°C et sous pression atmosphérique ;
(e) l'extrait de moringa est un extrait aqueux de moringa ; et
(f) l'huile de graines de moringa est différente de l'extrait de moringa.

2. Composition selon la revendication 1 dans laquelle l'huile de graines de moringa est extraite de graines d'au moins une espèce parmi *Moringa oleifera, Moringa pterygosperma, Moringa peregrina, Moringa concanensis* et *Moringa drouhardii.*

3. Composition selon la revendication 1 ou 2 dans laquelle l'huile de graines de moringa est présente en une quantité de 0,00001 à 1,5 %, de préférence de 0,004 à 0,3 % en poids de la composition.

4. Composition selon l'une quelconque des revendications précédentes dans laquelle l'extrait de moringa comprend une protéine, de préférence de 30 à 100 % en poids de l'extrait de moringa.

5. Composition selon l'une quelconque des revendications précédentes dans laquelle l'extrait de moringa porte le nom INCI *Moringa Pterygosperma* Seed Extract.

6. Composition selon l'une quelconque des revendications précédentes dans laquelle l'extrait de moringa est présent en une quantité de 0,000001 à 0,2 %, de préférence de 0,0002 à 0,01 % en poids de la composition.

7. Composition selon l'une quelconque des revendications précédentes dans laquelle le rapport pondéral de l'huile de graines de moringa à l'extrait de moringa est compris dans la plage allant de 18:1 à 45:1, de préférence de 24:1 à 45:1.

8. Composition selon l'une quelconque des revendications précédentes dans laquelle la composition peut comprendre de l'eau en une quantité de 25 à 78 % en poids de la composition.

9. Procédé de fourniture d'au moins un bénéfice choisi parmi un effet anti-âge, un effet antioxydant et une réduction du stress oxydatif comprenant une étape d'application de la composition de soins personnels selon l'une quelconque des revendications 1 à 8.

10. Utilisation d'une composition de soins personnels selon l'une quelconque des revendications 1 à 8 pour au moins un bénéfice choisi parmi un effet anti-âge, un effet antioxydant et une réduction du stress oxydatif.
